# EUROPÄISCHE PATENTANMELDUNG

(11) **EP 2 781 193 A1**
(43) Veröffentlichungstag der Anmeldung: **24.09.2014**
(21) Anmeldenummer: 14159684.1
(22) Anmeldetag: 14.03.2014
(51) Int. Cl.: A61B 17/02, A61B 17/34, A61M 25/06

(54) **Einführelement sowie Einführvorrichtung**

(30) Priorität: 18.03.2013 US 201361802759 P
(71) Anmelder: Biotronik AG, 8180 Bülach (CH)
(72) Erfinder: Schär, Louis, 3013 Bern (CH); Fargahi, Amir, 8180 Bülach (CH)
(74) Vertreter: Galander, Marcus

(57) **Zusammenfassung**

Die Erfindung betrifft ein Einführelement (100), welches zum Zusammenwirken mit einer Einführvorrichtung (200) zum Einführen eines medizinischen Implantats (222) in einen menschlichen oder tierischen Körper ausgebildet ist. Das Einführelement (100) umfasst eine Hülle (10) mit einem distalen Ende (40) und einem dem distalen Ende (40) gegenüberliegenden proximalen Ende (30), welche zumindest einen aufweitbaren Bereich (10a) aufweist, der aus einem ersten Zustand mit zumindest am proximalen Ende (30) kleinem lichten Querschnitt in einen zweiten Zustand mit zumindest am proximalen Ende (30) größerem lichten Querschnitt überführbar ist, wobei der aufweitbare Bereich (10a) der Hülle (10) im ersten Zustand flacher ausgebildet ist als im zweiten Zustand.

## Beschreibung

Die Erfindung betrifft ein Einführelement sowie eine Einführvorrichtung zum Einführen eines medizinischen Implantats in einen menschlichen oder tierischen Körper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina-Implantate, zahnmedizinische Implantate, Implantate zum Gelenkersatz, Gefäßprothesen oder Stents.

Implantate werden vor einem Einführen in den Körper mit Kathetern verbunden, mit dessen Hilfe sie am Einsatzort genau platziert und definiert freigegeben werden können. Zum Einschleusen in den tierischen und/oder menschlichen Körper wird hierzu ein schlauchartiges Einführelement eingesetzt, durch welches das Implantat mittels der Einführvorrichtung geschoben wird. Um die Belastung der Gefäße beim Einschleusen des Implantats zu vermindern, ist aus der US 2010/0094392 A1 ein Einführelement bekannt, welches erst beim Passieren des Implantats auf den notwendigen größeren Durchmesser aufgeweitet wird. Das Einführelement besteht aus zwei bis drei koaxial angeordneten Lagen, wobei die äußere mit longitudinalen Schlitzen versehen ist, was die Vergrößerung des Durchmessers ermöglicht.

Konventionelle Einführelemente sind jedoch häufig zur Anwendung bei kleinen Tieren zu groß.

Der Erfindung liegt die Aufgabe zugrunde, ein Einführelement anzugeben, das ein Einführen eines Implantats auch in Bereiche mit kleinen Abmessungen ermöglicht.

Eine weitere Aufgabe ist in der Bereitstellung einer entsprechenden Einführvorrichtung zu sehen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird ein Einführelement vorgeschlagen, welches zum Zusammenwirken mit einer Einführvorrichtung zum Einführen eines medizinischen Implantats in einen menschlichen oder tierischen Körper ausgebildet ist. Das Einführelement umfasst eine Hülle mit einem distalen Ende und einem dem distalen Ende gegenüberliegenden proximalen Ende, welche zumindest einen aufweitbaren Bereich aufweist, der aus einem ersten Zustand mit kleinem lichten Querschnitt zumindest am proximalen Ende in einen zweiten Zustand mit größerem lichten Querschnitt zumindest am proximalen Ende überführbar ist, wobei der aufweitbare Bereich der Hülle im ersten Zustand flacher ausgebildet ist als im zweiten Zustand.

Das proximale Ende ist bei bestimmungsgemäßer Handhabung des Einführelements einem Benutzer zugewandt, das distale Ende dem Implantat. Durch die Dehnbarkeit des Einführelements bei Durchführung des Implantats bzw. eines Katheters mit Implantat kann die Öffnung im Körper zur Einführung des Implantats kleiner ausfallen, als wenn eine Schleuse mit maximal benötigtem Durchmesser eingesetzt würde.

Das Einführelement weist zumindest in seinem aufweitbaren Bereich im ersten Zustand ein kleines, quasi zweidimensionales Profil auf und kann daher auch durch sehr kleine Schnitte oder Öffnungen geführt werden. Das Einführelement kann leicht vom ersten Zustand in den aufgeweiteten zweiten Zustand überführt werden und somit als Schleuse für eine Vielzahl von Durchmessern von Einführvorrichtungen, wie etwa Katheter, zum Einführen eines Implantats in den menschlichen oder tierischen Körper eingesetzt werden. Beispielsweise kann das Einführelement zur Stentimplantation bei kleinen Tieren, z.B. Mäusen, eingesetzt werden.

Unter einem Einführelement mit einem quasi zweidimensionalen Profil wird im Rahmen dieser Anmeldung verstanden, dass das Einführelement zumindest in seinem aufweitbaren Bereich im ersten Zustand eine Ebene mit maximaler Ausdehnung aufweist. Entlang einer beliebigen Achse senkrecht auf dieser Ebene ist die Ausdehnung höchstens das 0.5 fache, bevorzugt höchstens das 0.25 fache, besonders bevorzugt höchstens das 0.1 fache der maximalen Ausdehnung.

Nach einer günstigen Ausgestaltung kann der aufweitbare Bereich der Hülle des Einführelements vom ersten in den zweiten Zustand auffaltbar und/oder aufklappbar und/oder auffächerbar sein. Das Design des leicht auffaltbaren/aufklappbaren/auffächerbaren Einführelements ermöglicht die Kompatibilität des Einführelements mit vielen verschiedenen Katheterdurchmessern.

Nach einer günstigen Ausgestaltung kann die Hülle bzw. der aufweitbare Bereich der Hülle im ersten Zustand eine pfeilspitzenartige Kontur aufweisen, beispielsweise in Form eines Dreiecks. Andere Profile sind ebenfalls denkbar.

Gemäß einer günstigen Ausgestaltung kann die Hülle des Einführelements am distalen Ende eine lang ausgezogene Spitze aufweisen. Die Spitze erlaubt eine verbesserte Platzierung des Einführelements sowie der Einführvorrichtung beispielsweise in einem engen Gefäß. Die Spitze kann am aufweitbaren Bereich angesetzt sein, bzw. der aufweitbare Bereich kann in die Spitze übergehen. Dabei kann vorgesehen sein, dass die Spitze nicht aufgeweitet wird, wenn der aufweitbarer Bereich der Hülle beim Übergang vom ersten Zustand in den zweiten Zustand der Hülle aufgeweitet wird. Die Spitze kann eine Länge aufweisen, die einen signifikanten Anteil der Länge des aufweitbaren Bereichs beträgt bis hin zu einem Mehrfachen der Längserstreckung des aufweitbaren Bereichs. Beispielsweise kann die Länge der Spitze halb so lang sein wie der aufweitbare Bereich.

Gemäß einer günstigen Variante kann die Hülle eine Naht aufweisen, wobei beim Öffnen der Naht das Innere des aufweitbaren Bereichs freilegbar ist. Die Naht kann eine Sollbruchnaht sein, z.B. eine Naht mit Perforation, die leicht aufbrechbar ist. Die Naht erlaubt ein einfaches Entfernen des Einführelements nach dem Einführen der Einführvorrichtung, z.B. eines Katheters.

In vorteilhafter Ausgestaltung kann die Hülle im einführbaren Bereich, der als Schleuse für das medizinische Implantat vorgesehen ist, eine Wandstärke von höchstens 100 µm, bevorzugt höchstens 70 µm, besonders bevorzugt höchstens 50 µm, aufweisen. Dadurch ist eine besonders geringe Höhe des aufweitbaren Bereichs der Hülle möglich, wenn die Hülle im ersten, nicht-aufgeweiteten, flachen Zustand ist. Da das Profil im nicht-aufgeweiteten Zustand quasi "zweidimensional" ist, kann das Einführelement leicht in einen kleinen Schnitt eingeführt werden.

In einer günstigen Ausgestaltung kann die Hülle wenigstens bereichsweise aus einer Metallfolie mit einer Wandstärke von höchstens 100 µm, bevorzugt höchstens 70 µm, besonders bevorzugt höchstens 50 µm, gebildet sein. Eine Metallfolie ist mechanisch sehr stabil und kann besonders leicht z.B. gefaltet werden und aufgefaltet werden. Denkbar ist auch, dass die Hülle wenigstens bereichsweise aus einer Polymerfolie mit einer Wandstärke von höchstens 100 µm, bevorzugt höchstens 70 µm, besonders bevorzugt höchstens 50 µm, gebildet sein kann.

Vorteilhaft kann die gesamte Hülle die dünne Wandstärke aufweisen, womit ein einstückiges Einführelement auf einfache Weise aus einer entsprechenden Folie herstellbar ist. Geeignete Polymer-Materialien sind Polyether-ether-keton (PEEK), Polyimid (PI) Polyetherketon-keton (PEKK), Polyamide, Polyester, Polyethylen (PE), Polypropylen (PP), Polycarbonat (PC). Bevorzugt sind beispielsweise Polyamide z.B. Polyamid 12 (PA-12, bestehend aus Laurinlactam oder ω-Aminododecansäure) oder Polyester z.B. Polyethylenterephthalat, bekannt als PET. Ein geeignetes Metall ist z.B. Titan, Chirurgenstahl oder dergleichen.

In vorteilhafter Ausgestaltung kann die Hülle eine reibungsmindernde Beschichtung aufweisen. Diese kann auf der Innenseite vorgesehen sein, was das Einführen der Einführvorrichtung erleichtert, auf der Außenseite, was das Einführen des Einführelements in den Körper erleichtert und beidseits auf Außenseite und Innenseite vorgesehen sein. Die Beschichtung kann je nach Bedarf hydrophob oder hydrophil sein.

Nach einem weiteren Aspekt der Erfindung wird eine Einführvorrichtung zum Einführen eines medizinischen Implantats in einen menschlichen oder tierischen Körper vorgeschlagen, die zum Zusammenwirken mit einem erfindungsgemäßen Einführelement ausgebildet ist.

Durch das zweidimensionale, flache Profil des Einführelements kann die Einführbarkeit in den menschlichen oder tierischen Körper verbessert werden, so dass Komplikationen vermindert werden können. Das Miniatur-Einführelement erleichtert insbesondere die Forschung an neuen Implantaten in Tierversuchen.

Die Erfindung ist nachfolgend beispielhaft, anhand von in Zeichnungen dargestellten Ausführungsbeispielen, näher erläutert. Es zeigen in schematischer Darstellung:
- Fig. 1: eine Ansicht eines Einführelements nach einer Ausgestaltung der Erfindung in einem ersten, nicht-aufgeweiteten Zustand in quasi zweidimensionaler Form;
- Fig. 2: eine Ansicht des Einführelements aus Figur 1 beim Übergang aus dem ersten Zustand in einem zweiten, aufgeweiteten Zustand;
- Fig. 3: eine Ansicht des Einführelements aus Figur 1 im zweiten, aufgeweiteten Zustand mit durchgefädelter Einführvorrichtung;
- Fig. 4: eine Ansicht eines Einführelements nach einer weiteren Ausgestaltung der Erfindung in einem ersten, nicht-aufgeweiteten Zustand in quasi zweidimensionaler Form mit lang auslaufender Spitze am distalen Ende des Einführelements;
- Fig. 5: eine Ansicht eines Einführelements nach einer weiteren Ausgestaltung der Erfindung in einem ersten, nicht-aufgeweiteten Zustand in quasi zweidimensionaler Form mit einer Naht zum Entfernen des Einführelements von der Einführvorrichtung; und
- Fig. 6: eine Ansicht des Einführelements nach Figur 5 in mit geöffneter Naht.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

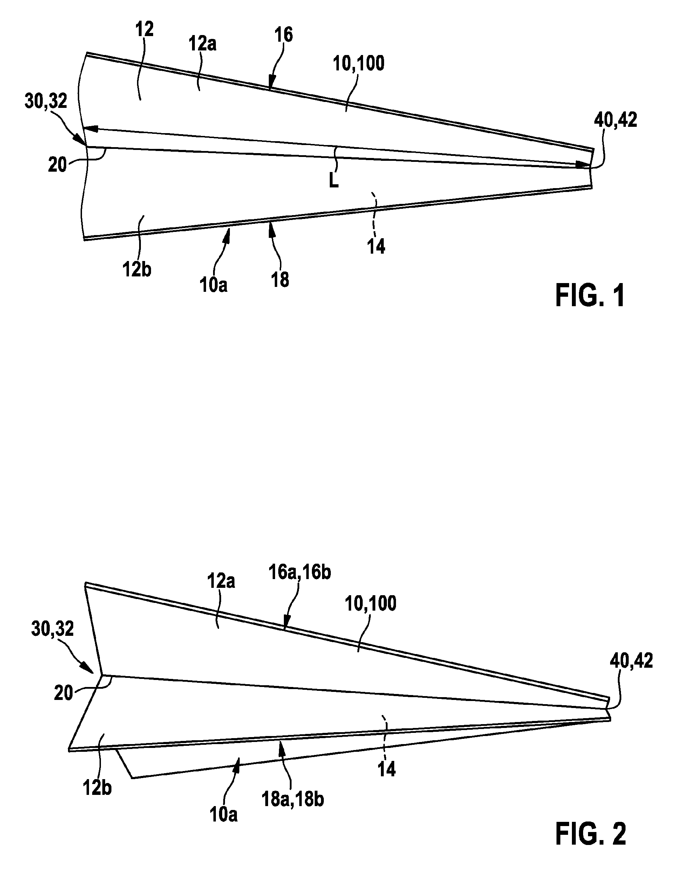

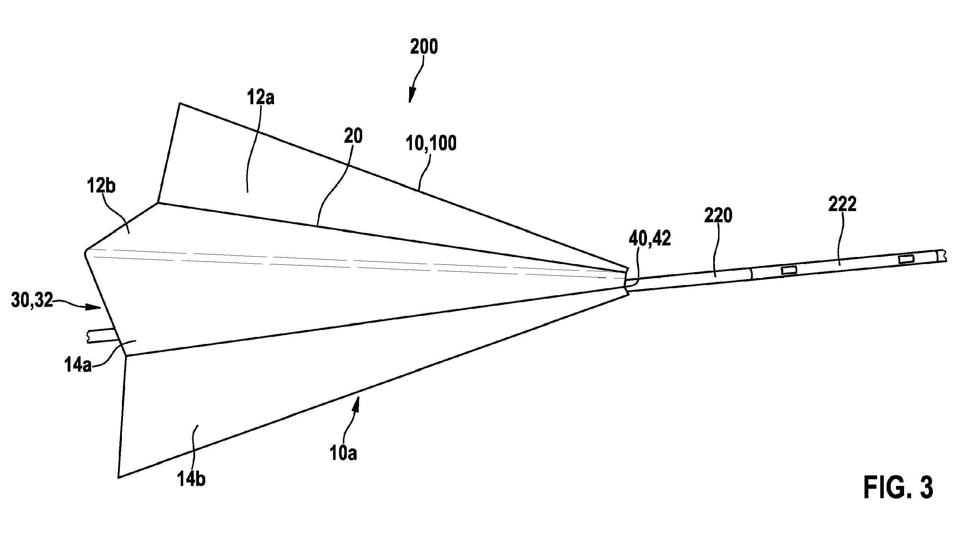

Die Figuren 1 bis 3 zeigen Ansichten eines Einführelements 100 nach einer Ausgestaltung der Erfindung in einem ersten, nicht-aufgeweiteten Zustand in quasi zweidimensionaler Form (Figur 1), beim Übergang aus dem ersten Zustand in einem zweiten, aufgeweiteten Zustand (Figur 2) und eine Anordnung 200 von Einführelement 100 im zweiten, aufgeweiteten Zustand und durch das Einführelement 100 durchgefädelter Einführvorrichtung 220 (Figur 3).

Das Einführelement 100 ist zum Zusammenwirken mit einer Einführvorrichtung 220 zum Einführen eines medizinischen Implantats in einen menschlichen oder tierischen Körper ausgebildet und bildet mit der Einführvorrichtung 220 eine Anordnung 200.

Das Einführelement weist eine Hülle 10 mit einem distalen Ende 40 und einem dem distalen Ende 40 gegenüber liegenden proximalen Ende 30 auf. Dabei weist die Hülle 10 in dieser Ausgestaltung einen aufweitbaren Bereich 10a auf. Dieser dient im bestimmungsgemäßen Einsatz als Schleuse für die Einführvorrichtung 220, die durch eine Öffnung 32 am proximalen Ende 30 und eine Öffnung 42 am distalen Ende 40 durch das Einführelement 100 führbar ist. In dieser Ausgestaltung bildet der aufweitbare Bereich 10a die Hülle 10.

Der lichte Querschnitt der Öffnung 32 am proximalen Ende 30 ist im ersten Zustand erheblich kleiner als im zweiten Zustand. Im ersten Zustand ist die Hülle 10 bzw. der aufweitbare Bereich 10a flach, quasi zweidimensional, ausgebildet. Die Hülle 10 weist im ersten Zustand eine pfeilspitzenartige Kontur auf, mit der Pfeilspitze am distalen Ende 40.

Entlang einer Längserstreckung L der Hülle 10 ist ein Falz 20 ausgebildet, wobei eine sichtbare Oberseite 12 aus einem ersten Flügel 12a auf der einen Seite des Falz 20 und einem zweiten Flügel 12b auf der anderen Seite des Falz 20 gebildet ist. Eine gegenüberliegende, in der Figur nicht sichtbare Rückseite 14, sowie zwischen der Vorderseite 12 und der Rückseite 14 eingeklappte Seiten 16, 18 sind vergleichbar ausgebildet. Aufgeklappt wird das Einführelement 100, bzw. die Hülle 10, indem die Flügel 12a, 12b der Vorderseite 12 und die der Rückseite an ihren Kanten nach außen gebogen und alle Falze 20 von der Mittellinie der Hülle 10 abgehoben werden, so dass sich als aufgeweiteter Zustand der Hülle 10 ein Kegel mit eckigem Querschnitt bildet.

Im aufgeweiteten Zustand (Figur 3) sind die Seitenflügel (von denen die Seitenflügel 12a, 12b, 14a, 14b zu erkennen sind) aufgefaltet und bilden einen um eine Mittelachse symmetrischen Körper, durch den die Einführvorrichtung 220 mit einem Implantat 222 durchgeführt werden kann.

Der einführbare Bereich der Hülle 10 dient als Schleuse für die Einführvorrichtung 220 mit dem medizinischen Implantat 222, durch welche die Einführvorrichtung in den menschlichen oder tierischen Körper eingeführt wird. Vorteilhaft ist die Wandstärke der Hülle 10 sehr gering, insbesondere deutlich unter 500 µm, höchstens 100 µm, bevorzugt höchstens 70 µm, besonders bevorzugt höchstens 50 µm, stark. Die Hülle 10 kann aus einer Metallfolie oder einer Polymerfolie gebildet sein und bei Bedarf eine reibungsmindernde Beschichtung aufweisen.

Die Einführvorrichtung 220 ist nicht im Detail dargestellt und kann in üblicher Weise einen Innenschaft umfassen, auf welchem das Implantat 222 gelagert ist, sowie einen Außenschaft, der Innenschaft mit Implantat 222 umschließt.

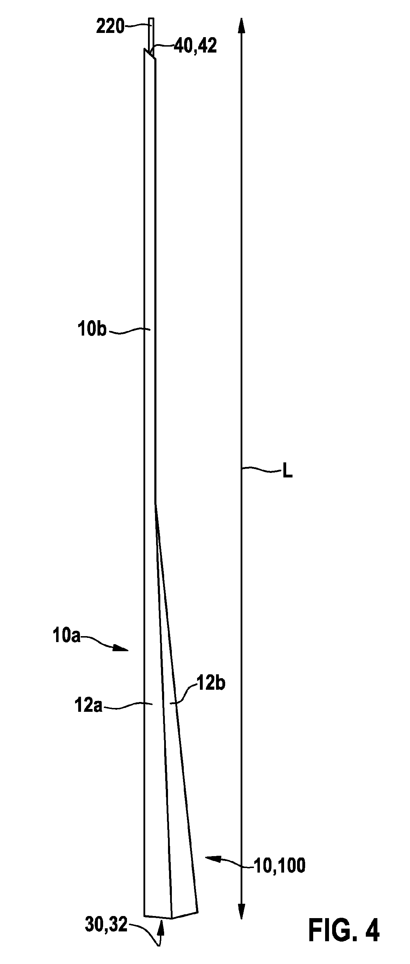

Figur 4 zeigt eine Ansicht eines Einführelements 100 nach einer weiteren Ausgestaltung der Erfindung in einem ersten, nicht-aufgeweiteten Zustand in quasi zweidimensionaler Form mit lang auslaufender Spitze 10b am distalen Ende 40 des Einführelements 100 und dessen nicht-aufweitbaren Bereichs 10a. Die Spitze 10b erlaubt eine verbesserte Führung von besonders dünnen Einführvorrichtungen 220. Dabei ist zweckmäßigerweise nur der aufweitbare Bereich 10a der Hülle 10, auch in diesem Beispiel mit pfeilspitzenartiger Kontur, aufweitbar, während die lang ausgezogene Spitze 10b nicht zwangsläufig ihren Durchmesser zwischen dem ersten Zustand und dem zweiten Zustand ändert. Die Hülle 10 kann aus einer einzigen Folie gefertigt sein, die an einem Ende gefaltet ist und den aufweitbaren Bereich 10a bildet und am anderen Ende die Spitze 10b.

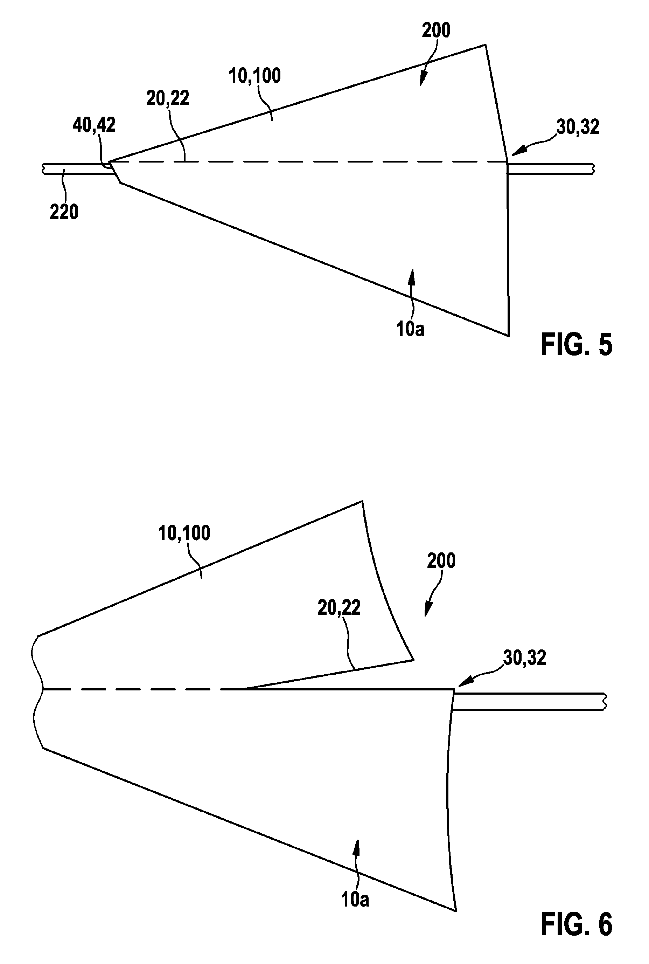

Die Figuren 5 und 6 zeigen eine weitere Ausgestaltung des Einführelements 100, wobei das Einführelement 100 eine Naht 22 entlang einer Längserstreckung L aufweist, die das Entfernen des Einführelements 100 von der Einführvorrichtung 220 erleichtert. Figur 6 zeigt dabei, wie die Naht 22 vom proximalen Ende 30 her geöffnet wird, um das Einführelement 100 von der Einführvorrichtung 220 abzuschälen. Die Naht 22 bildet dabei eine Sollbruchnaht in der Hülle 10.

Zum Einführen eines Katheters (Einführvorrichtung 220) in den menschlichen oder tierischen Körper wird dieser mit dem Implantat 220 voran durch das als Schleuse dienendes Einführelement 100 geschoben. Zur Einführung des Katheters wird zunächst die flexible Schleuse z.B. in ein Blutgefäß eingeführt, durch die der Katheter in das Blutgefäß gleiten kann. Durch Öffnen der Naht 22 (falls vorhanden) kann das Einführelement 100 bei Bedarf problemlos von der Einführvorrichtung 220 entfernt werden.

## Patentansprüche

1. Einführelement (100), welches zum Zusammenwirken mit einer Einführvorrichtung (220) zum Einführen eines medizinischen Implantats (222) in einen menschlichen oder tierischen Körper ausgebildet ist, **gekennzeichnet durch** eine Hülle (10) mit einem distalen Ende (40) und einem dem distalen Ende (40) gegenüberliegenden proximalen Ende (30), welche zumindest einen aufweitbaren Bereich (10a) aufweist, der aus einem ersten Zustand mit kleinem lichten Querschnitt zumindest am proximalen Ende (30) in einen zweiten Zustand mit größerem lichten Querschnitt zumindest am proximalen Ende (30) überführbar ist, wobei der aufweitbare Bereich (10a) der Hülle (10) im ersten Zustand flacher ausgebildet ist als im zweiten Zustand.

2. Einführelement nach Anspruch 1, **dadurch gekennzeichnet, dass** der aufweitbare Bereich (10a) der Hülle (10) vom ersten in den zweiten Zustand auffaltbar und/oder aufklappbar und/oder auffächerbar ist.

3. Einführelement nach Anspruch 1 oder 2, **dadurch gekennzeichnet, dass** der aufweitbare Bereich (10a) der Hülle (10) im ersten Zustand eine pfeilspitzenartige Kontur aufweist.

4. Einführelement nach einem der vorhergehenden Ansprüche, **dadurch gekennzeichnet, dass** die Hülle (10) am distalen Ende (40) eine lang ausgezogene Spitze (10b) aufweist.

5. Einführelement nach einem der vorhergehenden Ansprüche, **dadurch gekennzeichnet, dass** die Hülle (10) entlang der Längserstreckung (L) eine Naht (22) aufweist, wobei beim Öffnen der Naht (22) das Innere zumindest des aufweitbaren Bereichs (10a) freilegbar ist.

6. Einführelement nach einem der vorhergehenden Ansprüche, **dadurch gekennzeichnet, dass** die Hülle (10) im einführbaren Bereich, der als Schleuse für das medizinische Implantat (222) vorgesehen ist, eine Wandstärke von höchstens 100 µm, bevorzugt höchstens 70 µm, besonders bevorzugt höchstens 50 µm, ausweist.

7. Einführelement nach einem der vorhergehenden Ansprüche, **dadurch gekennzeichnet, dass** die Hülle (10) wenigstens bereichsweise aus einer Metallfolie mit einer Wandstärke von höchstens 100 µm, bevorzugt höchstens 70 µm, besonders bevorzugt höchstens 50 µm, gebildet ist.

8. Einführelement nach einem der vorhergehenden Ansprüche, **dadurch gekennzeichnet, dass** die Hülle (10) wenigstens bereichsweise aus einer Polymerfolie mit einer Wandstärke von höchstens 100 µm, bevorzugt höchstens 70 µm, besonders bevorzugt höchstens 50 µm, gebildet ist.

9. Einführelement nach einem der vorhergehenden Ansprüche, **dadurch gekennzeichnet, dass** die Hülle (10) eine reibungsmindernde Beschichtung aufweist.

10. Einführvorrichtung (220) zum Einführen eines medizinischen Implantats (222) in einen menschlichen oder tierischen Körper, die zum Zusammenwirken mit einem Einführelement (100) nach einem der vorhergehenden Ansprüche ausgebildet ist.
